Europäisches Patentamt

European Patent Office

Office européen des brevets

(19)

(11) Publication number: **0 272 791**
**A2**

# EUROPEAN PATENT APPLICATION

(12)

(21) Application number: **87310101.8**

(22) Date of filing: **16.11.87**

(51) Int. Cl.4: **C12Q 1/18**

(30) Priority: **17.11.86 US 932154**

(43) Date of publication of application:
**29.06.88 Bulletin 88/26**

(84) Designated Contracting States:
**AT BE CH DE ES FR GB GR IT LI LU NL SE**

(71) Applicant: **ONCOTECH**
**1791 Kaiser Avenue**
**Irvine California 92714(US)**

(72) Inventor: **Weisenthal, Larry M.**
**17031 Courtney Lane**
**Huntington Beach California 92649(US)**

(74) Representative: **Goldin, Douglas Michael et al**
**J.A. KEMP & CO. 14, South Square Gray's Inn**
**London WC1R 5EU(GB)**

(54) **Method to monitor and circumvent acquired resistance to chemotherapy in humans.**

(57) Acquisition of drug resistance of tumor cells in patients subjected to chemotherapeutic treatment is monitored by periodically withdrawing fresh tumor cells from the patient and determining the in vitro sensitivity of the withdrawn cells to the administered drug. This determination can also be used to diagnose relapse in a patient receiving chemotherapy. By making repeated withdrawals of tumor cells and repeated determinations of in vitro sensitivity of the withdrawn cells to the administered drug and by comparing the sensitivities determined, chemotherapeutic protocols can be modified.

EP 0 272 791 A2

# METHOD TO MONITOR AND CIRCUMVENT ACQUIRED RESISTANCE TO CHEMOTHERAPY IN HUMANS

## Field of the Invention

The invention relates to monitoring therapeutic treatment by in vitro diagnosis. In particular. it relates to using in vitro tests to follow the acquisition of resistance to chemotherapy in human tumors and to methods to assess alternative treatment regimes.

## Background of the Invention

The use of chemotherapy in treatment of human tumors is widespread, but notoriously unsuccessful. One of the reasons in a large number of cases appears to be the acquisition of resistance to the compounds selected for the chemotherapy on the part of some fraction of the tumor cell population. In a large proportion of individuals, initial successful remission is followed by relapse, presumably due to this alteration in the tumor metabolism, although this has not been established directly. Studies using established cell lines and animal tumors, however. have permitted some understanding of the mechanisms of drug resistance in these cases (Chabner. B.A., et al, Proc Am Assoc Cancer Research (1985) 26 :390-391). However, in human patients, most frequently the acquisition of drug resistance on the part of the tumor has simply been presumed from the pattern of response in the individual patient.

It is also known that human tumors are highly variable and that a drug regime workable for one patient may be totally ineffective in another. Efforts have been made to study the effects of drugs on individual human tumors, including solid tumors and hematologic neoplasms using in vitro laboratory tests. Many of these assays are clonogenic in nature, i.e., rely on the ability to culture and grow the neoplasms in vitro, which is often difficult.

An in vitro assay which circumvents the necessity for tumor growth in culture has been reported. (Weisenthal, L.M., et al, Cancer Research (1983) 43:258-264, 749-757.) This assay, further described below as readily adaptable to the method of the invention, relies on the ability of living cells to exclude certain dyes, whereas dead cells do not. The number of individual cells capable (or not) of dye uptake is ascertained by counting populations using a microscope. Thus the assay provides a means for individually counting the number of tumor cells surviving in the presence or absence of drug when the biopsied tumor cells are subjected to the assay, and has been used to predict the chemosensitivity of human tumors to various drugs (Weisenthal, L.M., et al, Cancer (1983) 51:1490-1495). A review of the development and relevance of this assay is set forth in Weisenthal, L.M., et al in Recent Results in Cancer Research (1984) 94:161-173, Springer Verlag, Berlin-Heidelberg.

Correlation of in vitro results in drug sensitivity tests to in vivo performance is generally difficult, but attempts have been made to do so (Bosanquet, A.G., et al, Brit J Cancer (1983) 47:781-789; Bird, M.C., Brit J Cancer (1986) 53:539-545). It would be extremely useful to have a method whereby in vitro monitoring of patients for acquisition of drug resistance to chemotherapeutic regimes and for designing modification to these regimes to accommodate the drug resistance. Such a method is provided by the invention herein.

## Disclosure of the Invention

The invention provides a method whereby individual human patients can be monitored for acquisition of resistance to the drugs administered in their particular chemotherapy programs, and whereby alternative programs to which the tumors are still sensitive can be designed. The method is based on withdrawal of fresh tumor cell samples from the patient and assessing the tumor cells in vitro for short term sensitivity to the drugs utilized in the chemotherapeutic regime. If resistance is found, the assay can be used to predict the effects of modifying substances which may render the previously administered drugs effective, or to predict the sensitivity of the patients' cells to alternative chemotherapeutic compounds.

Therefore, in one aspect, the invention relates to a method of monitoring acquisition of drug resistance in patients subject to chemotherapeutic treatment, which method comprises
periodically over the time the chemotherapeutic treatment is administered withdrawing a sample containing fresh tumor cells from said subject, and
determining in vitro the sensitivity of said cells to the administered drug.

In another aspect, the invention relates to the foregoing method, which further includes assessment of alternative drug protocols. Thus, in this other aspect, the invention relates to a method to successively modify a chemotherapeutic protocol in a human subject which comprises

periodically withdrawing a sample of fresh tumor cells from the subject:

determining the sensitivity of said cells to the drugs administered:

comparing the sensitivity of the most recently withdrawn sample to that of previous samples: and if diminished sensitivity is found, withdrawing additional fresh tumor cells, and

determining the sensitivity of said cells to alternative drugs.

The invention is also directed to a single withdrawal of fresh cells after the fact of chemotherapy to ascertain by in vitro tests, whether the tumor cells have acquired drug resistance.

## Brief Description of the Drawings

Figure 1 shows prospective clinical correlations in human lymphatic neoplasms.

Figures 2 and 3 show summaries of correlations between drug resistance in previously treated and untreated patients.

## Modes of Carrying Out the Invention

The invention relies on an in vitro assay procedures and requires the withdrawal of fresh tumor cells from patients being subjected to chemotherapy in order to evaluate the continued effectiveness of the drug. Preferably the tumor cell withdrawal is performed periodically to monitor, continuously, the progress of the therapy. In addition, for those patients where increased resistance to the drug regime is found, fresh tumor samples are evaluated using an in vitro assay for their sensitivity either to the same drug in combination with some modifying agent, or to a new drug entirely. By such redesign, the treatment of the patient can be modified to accommodate the resistance acquired.

## General Methods

A convenient in vitro assay is the differential staining cytotoxicity assay (DiSC) as described in Weisenthal, L.M., et al, Cancer Research (1983) ( supra) and as modified by Bird et al (Bird, M.C., et al. Hematol Oncol (1985) 3:1-9). Briefly, the method is as follows:

Bone marrow specimens, peripheral blood buffy coats (approximately 1 cm$^3$), or other appropriate tumor cell specimens are diluted with 6-10 volumes of complete RPMI-1640 medium containing 10 units/ml of heparin. This is underlayered with Ficoll-diatrizoate (Lymphocyte Separation Medium, Litton Bionetics, Kensington, MD) and centrifuged. The interface layer is washed and counted. Solid tumors are manually teased apart after incubation with collagenase/DNAse. Cells (0.1-1 x 10$^6$, depending on cell yield) are aliquoted into 4-ml capacity conical polypropylene tubes and brought to a volume of 0.9 ml with complete RPMI-1640 medium plus 10% heat-inactivated fetal calf serum: 0.1 ml of 10x drug solution is added to the cell suspensions, and cells are incubated with drugs for either 1 hour (mechlorethamine, doxorubicin, melphalan, and carmustine) or continuously (other drugs).

Afte 4 days in culture, 0.2 ml of a suspension of acetaldehyde-fixed duck red blood cells (DRBC), containing 50,000 DRBC, is added to each tube, and the cells are concentrated to a final volume of 0.2 ml by centrifugation. Following this, 0.2 ml of Fast Green (solid tumors) or Fast Green-Nigrosin (hematologic neoplasms) (1.0% Fast Green and 0.5% Nigrosin in 0.15 M NaCl) is added and the tubes are vortexed. After 10 minutes, cell suspensions are again vortexed and cytocentrifuged (1200 rpm on a Cytospin I or 1080 rpm on a Cytospin II) for 8 minutes. The cell disks are than counterstained with hematoxylin and eosin (h&E) (solid tumors) or are fixed with methanol for 20 seconds and counterstained with Wright-Giemsa (hematologic neoplasms) in an automated slide stainer (Ames Hematek). Mounting balsam (Histomount, National Diagnostics, Somerville, NJ) and coverslips are added. Slides are then counted at either 400x or 1000x on a standard light microscope by a trained hematology technologist. "Living" cells stained with H&E or Wright-Giemsa. "Dead" cells stained green with Fast Green and black or black-green with Fast Green-Nigrosin. DRBC stain green and are easily identified as nucleated microelliptocytes. Only "living" tumor cells and DRBC are counted. The "living" tumor cells/DBRC ratio is determined, and the ratios from drug-treated cultures are compared with the ratios from control cultures (cultures containing drug vehicle, usually 0.9% NaCl) and expressed as percent of control.

While the invention is not limited to use of the specific foregoing in vitro assay method, this method has been shown to be highly reliable and useful. A large percentage of assays performed using this method are "successful".

A "successful" assay is defined as an assay in which at least 100 viable and recognizable neoplastic cells were present on the control slides and in which a result could be obtained for three or more drugs. In practice, dozens of drugs can be tested in a typical assay.

Success rates obtained by Applicant were (successful assays/specimens received): CLL.60/66 (91%); multiple myeloma, 23/30 (77%); non-Hodgkin's lymphoma, 14/19 (74%); ALL, 37 45 (82%); CML-blast crisis, 13/14 (93%).

The most common reason for a failed assay is poor survival of the neoplastic cells in the control culture or insufficient percentage of neoplastic cells in the specimen received. In general, a successful assay would require a specimen with > 15% plasma cells (for myeloma), > 30% blasts (for acute leukemia), and> 80% lymphocytes(for CLL). It has been found that with nearly equal success rates, standard EDTA blood specimens "leftover" in the clinical hematology laboratory (which would be otherwise discarded) after analysis on the Coulter Counter can be utilized. Surprisingly, contamination was only rarely a problem with these specimens. Additionally, specimens may be shipped via overnight mail, so long as adequate thermal protection is provided.

The drugs used in chemotherapy are generally known in the art and include, for example, mechlorethamine, melphalan, carmustine, cytarabine dexamethasone, doxorubicin, cisplatin, vincristine. Drug concentrations and times of exposure in the assay are determined individually. Some drugs can be tested at a 1-hour exposure; however certain drugs (e.g., dexamethasone, cytarabine, and vincristine) are not active at a 1-hour exposure in the assay and must be tested at a continuous exposure. Thus, the assay should be calibrated for different drugs with either a 1-hour or a continuous exposure. Continuous exposures are unquestionably simpler and the assay can be calibrated on the basis of a continuous exposure for all drugs.

Sample preparation depends on the nature of the sample being taken, and follows standard guidelines for obtaining specimens for human studies. For example, depending on the nature of the tumor, specimens may include EDTA-anticoagulated blood or marrow specimens and solid tumor biopsy specimens. Solid tumors are collected in complete tissue culture medium with antibiotics.

In the results set forth below, the in vivo data for correlation were obtained by evaluation of the patients by their primary physicians according to standard criteria, which included 50% reduction in the product of perpendicular diameters of lymph nodes (chronic lymphocytic leukemia (CLL) lymphoma), 50% reduction in serum myeloma protein (multiple myeloma), and reversion to chronic myelocytic leukemia (CML) in Blass crisis CML. Response in acute lymphoblastic leukemia (ALL) was defined as complete remission.

Effective use of in vitro information in detecting drug resistance acquisition and in designing therapy requires that correlation of in vitro results from the protocol selected with in vivo response be established. Validation of protocols, such as that of the invention, which are useful in predicting in vivo efficacy is absolutely essential, as the outcome of correlation studies is unpredictable and uncertain. While a general theoreticial principle may be in place which would provide a rationale to attempt the use of a laboratory protocol, this is no guarantee or even substantial probability that a positive correlation will result.

The results below provide validation of the method of the invention.

## Results

Statistical correlations were established by standard methods in these results.

Table 1 shows the general in vitro activity of a number of drugs in specimens of human neoplasms.

TABLE 1.—In vitro "phase II trials": in vitro activity of various drugs in cultures of human leukemias and solid tumors*

| Neoplasm | Dexamethasone | Cytarabine | Cisplatin | Vincristine | Mechlorethamine | Doxorubicin |
|---|---|---|---|---|---|---|
| Assorted solid tumors | 0% (34) | ?% (31) | — | — | — | — |
| ALL | 61% (33) | 68% (19) | — | 56% (25) | 80% (20) | 72% (18) |
| ANLL | 7% (41) | 76% (34) | — | 22% (23) | 56% (16) | 48% (27) |
| CLL | 28% (43) | — | 0% (16) | 47% (15) | 67% (33) | 56% (30) |
| Small Cell Lung Cancer | — | — | 71% (14) | 50% (10) | 67% (21) | 63% (19) |
| Non-Small Cell Lung Cancer | — | — | 35% (66) | 8% (48) | 14% (77) | 4% (81) |

*% of all specimens tested which were "sensitive" in vitro (< 30% cell survival) to dexamethasone (0.4 μg/ml), cytarabine (3.3 μg/ml), cisplatin (3.3 μg/ml) and vincristine (0.26 μg/ml), all tested at a continuous, 4-day exposure. Mechlorethamine (3.5 μg/ml) and doxorubicin (1.67 μg/ml for ALL and ANLL; 1.2 μg/ml for CLL and small cell and non-small cell lung cancers) were tested at a 1-hour exposure. Values in parentheses = Nos. of different specimens of each type assayed at each drug exposure. Dashes indicate that an inadequate number of specimens were tested at that particular drug exposure. Approximately two-thirds of the specimens from each type of leukemia were from previously untreated patients, while approximately 90% of the solid tumor specimens were from previously untreated patients.

These specimens were obtained from both previously untreated and previously treated patients, similar to the situation existing in a clinical phase II trial. Dexamethasone was "active (< 30% cell survival) in vitro in the majority of ALL specimens but only minimally active in acute non-lymphoblastic leukemia (ANLL) and not active in solid tumor specimens. Cytarabine was active in both ALL and ANLL but not in solid tumors. Non-small cell lung cancer was found to be generally resistant to all drugs, while the most active agent tested was cisplatin. In contrast, small cell lung cancer was highly sensitive to the same agents. ALL and ANLL were sensitive to the drugs tested. ALL was somewhat more sensitive than ANLL to doxorubicin and considerably more sensitive to vincristine. CLL was highly sensitive to most of these agents. One might wonder if this was a nonspecific sensitivity to toxic agents. However, CLL was completely refractory to the same concentration of cisplatin that had high activity in small cell lung cancer and moderate activity in non-small cell lung cancer. These in vitro results correspond closely to the known clinical activities of these

5

agents.

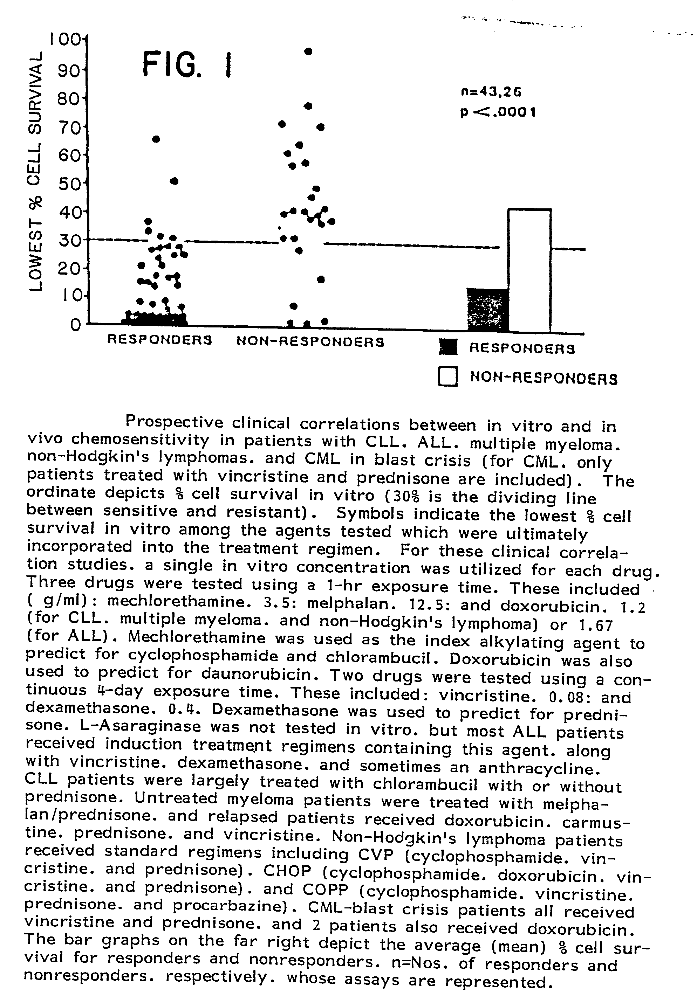

Figure 1 shows prospective clinical correlations in human lymphatic neoplasms. We have used the convention of placing a 30% cell survival as the cutoff between sensitive and resistant. Single agents were tested in vitro, but almost all patients were treated with drug combinations. We have based our predictions on the activity of the most active of the single agents tested in vitro (i.e. the agent producing the least cell survival or greatest cell kill). There were clear correlations between in vitro and in vivo results. Of the patients who responded to clinical chemotherapy, 37 of 43 had previously had at least one of the drugs used in their treatment regimens shown to be in the sensitive range (<30% cell survival). In Figure 1, the bar graphs on the right show the average percent cell survival for the responders (solid bars) versus the nonresponders (open bars). The average in vitro cell survival of the responders was significantly less than that of the nonresponders. A summary of these prospective clinical correlations is shown by disease type in Table 2.

TABLE 2.—Prospective clinical correlations in human lymphatic neoplasms*

| Neoplasm | True positives | False positives | True negatives | False negatives |
|---|---|---|---|---|
| CLL* | 9 | 1 | 4 | 1 |
| Multiple myeloma | 2 | 1 | 2 | 1 |
| Non-Hodgkin's lymphoma | 6 | 1 | 3 | 0 |
| ALL | 18 | 2 | 5 | 4 |
| CML-blast crisis† | 2 | 1 | 6 | 0 |
| Totals‡ | 37 | 6 | 20 | 6 |

*Data depict the Nos. of true positives (sensitive in vitro, responsed to chemotherapy in vivo), false positives (sensitive in vitro, no response to chemotherapy in vivo), true negatives (resistant in vitro, no response to chemotherapy in vivo), and false negatives (resistant in vitro, response to chemotherapy in vivo). The mix of previously untreated and previously treated patients in each group consisted of: CLL, 11 untreated and 4 treated; multiple myeloma, 3 untreated and 3 treated; non-Hodgkin's lymphoma, 4 untreated and 6 treated; ALL, 21 untreated and 8 treated; and CML, 8 untreated and 1 treated.
†Treated with vincristine + prednisone only.
‡$P < 0.0001$.

It must be noted that these data describe a heterogeneous population previously treated and untreated patients, as noted in the legend to Table 2. Additionally, in a number of cases, patients received treatment with some agents that were not tested in vitro. For example, most ALL patients received L-asparaginase, but this drug was not assayed in vitro. This incomplete testing would tend to have the effect of biasing the results in the direction of fewer true positives, more false negatives, more true negatives, and fewer false positives. In other words, there is a bias for a lower sensitivity and a higher specificity. In the 37 "true positive" cases, cells were tested with an average of 2.0 (±0.96 SD) drugs (including only tests of those drugs with which patients were subsequently treated). These same patients actually received treatment with an average of 2.8 (±1.2) drugs. In the six "false negative" cases, cells were tested with an average of 1.8 (±0.75) drugs, but patients were treated with an average of 3.0 (±0.89) drugs. In the 19 "true negative" cases, cells were tested with an average of 2.0 (± 0.86) drugs, but patients were treated with an average of 2.4 (±1.0) drugs. In the six "false positive" cases, cells were tested with an average of 2.5 (±1.0) drugs, but patients were treated with an average of 3.0 (±1.4) drugs. Thus, our results were comparatively more biased to give a lower sensitivity than they were biased to give a higher specificity.

Because of the potential biases, we also evaluated different subsets of assays. First, considering only cases of previously untreated patients, there were 29 true positives, three false positives, ten true negatives, and five false negatives. Next, considering only cases of previously treated patients, there were eight true positives, three false positives, ten true negatives, and one false negative. (The higher true positive rate in untreated patients and the higher true negative rate in treated patients are mathematically expected in sets of patients with different response probabilities). Next we included only assays in which cells were tested against all drugs with which the patients were subsequently treated. In this case, there were 17 true positives, four false positives, 13 true negatives, and two false negatives. Considering only cases in which all drugs were tested in cells from previously untreated patients, there were 12 true positives, two false positives, six true negatives, and one false negative. Finally, considering only cases in which all drugs were tested in cells from previously treated patients, there were five true positives, two false positives, seven true

6

negatives, and one false negative. Thus, despite the limitations of testing single agents in cells from patients treated with combinations, in vitro chemosensitivity did appear to correlate with clinical response, in all subsets of patients.

Next we looked at previously untreated versus previously treated patients. These data and the metachronous assay data (to follow) are germane both to the validity of the assay as it relates to prediction of drug efficacy and to the validity of the assay protocol of the invention as it pertains to monitoring acquired drug resistance.

## Validity of the Assay to Detect Drug Resistance

Comparison of assay results on freshly drawn tumor cells performed on treated and untreated patients shows that drug resistance found in the assay in treated patients correlates with relapse in vivo.

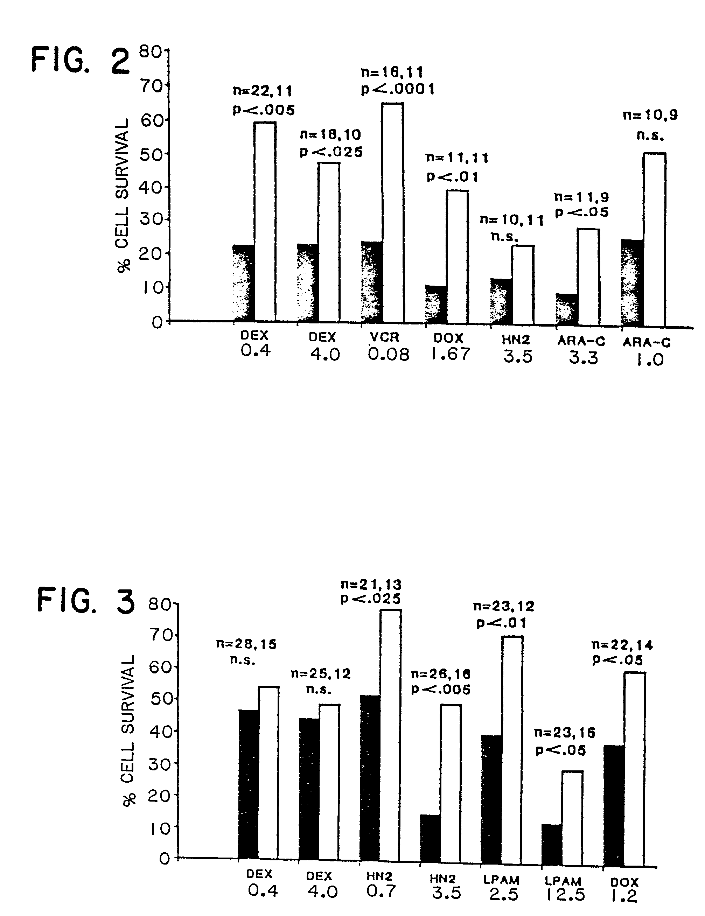

Figure 2 and Table 3 show summaries of assays performed on patients with ALL. Figure 2 shows graphically the data of Table 3, which demonstrate that treated patients, in general, show higher drug resistance in this in vitro protocol than do untreated patients.

TABLE 3.—Sensitivity vs resistance in vitro: ALL

| Drug | Concentration ($\mu$g/ml) | Untreated patients* | | Treated patients* | | P value† |
|---|---|---|---|---|---|---|
| | | Sensitive | Resistant | Sensitive | Resistant | |
| Dexamethasone | 0.4 | 16 | 6 | 4 | 7 | 0.043 |
| Dexamethasone | 4.0 | 13 | 5 | 3 | 7 | 0.034 |
| Vincristine | 0.08 | 10 | 6 | 1 | 10 | 0.0068 |
| Doxorubicin | 1.67 | 10 | 1 | 4 | 7 | 0.011 |
| Mechlorethamine | 3.5 | 8 | 2 | 9 | 2 | 0.41 |
| Cytarabine | 1.0 | 6 | 4 | 2 | 7 | 0.10 |
| Cytarabine | 3.3 | 9 | 2 | 5 | 4 | 0.18 |

*Values = No. of patients. Sensitive: < 30% cell survival: resistant: > 30% cell survival. Drugs tested using a continuous, 4-day exposure included dexamethasone, 0.4 and 4.0 $\mu$g/ml: vincristine, 0.08: and cytarabine, 3.3 and 1.0. Drugs tested using a 1-hr exposure included doxorubicin, 1.67 $\mu$g/ml and mechlorethamine, 3.5.
† Fisher exact test.

The relapsed patients had, for the most part, received therapy with vincristine, prednisone, L-asparaginase, and an anthracycline. Several patients had received cyclophosphamide at some point, although usually not in high doses or for an extended time period. Most treated patients did not receive cytarabine, except intrathecally. Relapsed patients were significantly more resistant in the assay to vincristine, dexamethasone, doxorubicin, and the higher concentration of cytarabine. Cells from relapsed patients were not significantly more resistant to mechlorethamine, our index alkylating agent (tested because cyclophosphamide is not active in vitro).

Different patterns of resistance were found for CLL specimens from patients who had received prior treatment with alkylating agents (usually chlorambucil or cyclophosphamide) with or without prednisone. These specimens were significantly more resistant to alkylating agents than were the specimens from previously untreated patients as shown in Figure 3 and Table 4. The specimens were also significantly more resistant to doxorubicin, even though the patients had not received treatment with this drug.

TABLE 4.—Sensitivity vs resistance in vitro: CLL

| Drug | Concentration (μg/ml) | Untreated patients* | | Treated patients* | | P value† |
|---|---|---|---|---|---|---|
| | | Sensitive | Resistant | Sensitive | Resistant | |
| Dexamethasone | 0.4 | 10 | 18 | 2 | 13 | 0.09 |
| Dexamethasone | 4.0 | 10 | 15 | 2 | 10 | 0.12 |
| Mechlorethamine | 0.7 | 5 | 16 | 1 | 12 | 0.20 |
| Mechlorethamine | 3.5 | 22 | 4 | 6 | 10 | 0.0023 |
| Melphalan | 2.5 | 8 | 15 | 1 | 11 | 0.08 |
| Melphalan | 12.5 | 20 | 3 | 8 | 8 | 0.014 |
| Doxorubicin | 1.2 | 11 | 11 | 4 | 10 | 0.13 |

*Values = No. of patients. Sensitive: < 30% cell survival; resistant: > 30% cell survival. See footnote (*) to table 3. Melphalan was tested using a 1-hr exposure.
†Fisher exact test.

A similar pattern was seen in assays performed on specimens from patients with non-Hodgkin's lymphoma, multiple myeloma, and blast crisis CML (considered together, as patient numbers were insufficient for separate analysis by disease type). Specimens from previously treated patients were significantly more resistant in vitro to alkylating agents and doxorubicin than were specimens from previously untreated patients (data not shown).

Metachronous Assays: Validity of the Method to Monitor Drug Resistance Acquisition

For monitoring drug resistance acquisition according to the method of the invention, fresh tumor cells are withdrawn at intervals and assayed in vitro. The results of the metachronous assays show that in vitro resistance tracks in vivo symptomology, and that patients not receiving drug therapy retained the original sensitivity/resistance patterns in vitro for their tumor cells.

We were able to perform two to four different assays on specimens obtained at intervals of between 3 days and 21 months from 11 different patients who did not receive intervening chemotherapy between two consecutive assays. Sixteen different pairs of metachronous assays could be analyzed from the 11 patients. Metachronous comparisons of the effects of identical drug concentrations tested in the assay could be made in 104 instances. Likewise, we were abe to perform two to four different assays on specimens obtained at intervals of between 10 days and 32 months from 18 different patients who did receive intervening chemotherapy between two consecutive assays. Twenty-five different pairs of metachronous assays could be analyzed from the 18 patients. Metachronous comparisons of the effects of identical drug concentrations tested in the assay could be made in 114 instances. Tables 5 and 6 show the results of metachronous assays performed in patients who either had not received or had received intervening chemotherapy.

TABLE 5.—Metachronous assays: no intervening therapy[a]

| Patient code | Date (mo/day/yr) | Dexamethasone | | | Mechlorethamine | | | Melphalan | | | Carmustine | Vincristine | Doxorubicin |
|---|---|---|---|---|---|---|---|---|---|---|---|---|---|
| | | 0.4 | 1.3 | 4.0 | 0.7 | 1.2 | 3.5 | 2.5 | 4.2 | 12.5 | 11.0 | 0.08 | 1.2 |
| CLL-A | 05/15/85 | 43 | | 42 | | | | | | | | | |
| CLL-A | 06/04/85 | 77 | | 87 | | | | | | | | | |
| CLL-A | 07/22/85 | 42 | | 40 | | | 95 | | | | | 46 | |
| CLL-A | 08/08/85 | 50 | | 55 | | | 103 | | | | | 48 | |
| CLL-D | 05/16/85 | | 3 | 2 | 114 | 121 | 107 | 125 | 88 | 91 | - | | |
| CLL-D | 06/03/85 | | 24 | 11 | 61 | 84 | 35 | 48 | 75 | 44 | | | |
| CLL-E | 10/18/83 | 49 | 36 | 47 | 51 | | 5 | | | | | | |
| CLL-E | 10/31/83 | 44 | 33 | 39 | 80 | | 28 | | | | | | |
| CLL-F | 09/02/83 | 52 | | 41 | 87 | | 34 | 94 | | 49 | 118 | | 65 |
| CLL-F | 03/02/84 | 78 | | 73 | 92 | | 42 | 78 | | 34 | 69 | | 70 |
| CLL-G | 10/17/83 | 29 | | 21 | 54 | 15 | 17 | 28 | 11 | 5 | 58 | | 28 |
| CLL-G | 11/16/83 | 113 | 53 | 72 | 44 | 30 | 0 | 33 | 6 | 0 | 40 | | 12 |
| CLL-G | 02/28/84 | 84 | 82 | 83 | 46 | 5 | 0 | 29 | 12 | 0 | 77 | | 2 |
| CLL-G | 04/05/84 | 123 | 95 | 104 | 44 | 10 | 0 | 45 | 3 | 0 | 87 | | 4 |
| CLL-H | 06/02/83 | 50 | 39 | 20 | 37 | | 9 | 44 | | 15 | | | |
| CLL-H | 11/10/83 | 31 | 32 | 36 | 82 | 50 | 9 | 51 | 81 | 22 | | | |
| CLL-H | 07/27/84 | 64 | | | 45 | 47 | 2 | 64 | 63 | 8 | | | |
| CLL-I | 09/22/82 | 13 | 10 | 12 | | | | | | | | | |
| CLL-I | 10/04/82 | 6 | 5 | 5 | | | | | | | | | |
| CLL-L | 09/09/83 | 62 | | 56 | 62 | | 15 | 29 | | 4 | 47 | | 19 |
| CLL-L | 06/04/85 | 63 | | 55 | 69 | | 1 | 10 | | 1 | 19 | | 7 |
| MM-B | 11/04/80 | 135 | | | | 40 | 78 | 60 | 47 | 3 | 15 | 0 | 42 |
| MM-B | 11/12/80 | 73 | | | | 55 | 25 | 80 | 54 | 8 | 22 | 0 | 85 |
| ALL-F | 01/08/81 | 135 | 130 | 126 | | 0 | 5 | | | | | | 1 |
| ALL-F | 01/12/81 | 91 | 95 | 87 | | 2 | 8 | | | | | | 9 |
| ALL-C | 07/25/85 | 54 | | 46 | | | | | | | | 44 | |
| ALL-C | 07/28/85 | 85 | | 79 | | | | | | | | 71 | |

[a] Values = average % cell survival in vitro from assays on specimens from patients with lymphatic neoplasms who were assayed on 2 separate occasions. Only results from drugs assayed at an identical concentration and exposure time during the 1st and 2nd assay are included. Drugs included in this analysis are indicated, with drug concentrations listed in $\mu g/ml$. Assay results for each drug concentration are expressed as % cell survival (100% survival = 0% cell kill). Drug exposure durations were either 1 hour or continuous, as described in the Methods section. Means and comparisons of significance are described in the text. Significance levels are based on paired t test calculations from the individual paired comparisons of the 1st and 2nd assays in specimens from patients who had no intervening chemotherapy between the 1st and 2nd assays. Assays are further analyzed in the text to include (a) all assays or (b) only assays which were initially in the sensitive range (< 30% cell survival) at the time of the first assay.

TABLE 6.—Metachronous assays: intervening therapy[*]

| Patient Code | Date (mo/day/yr) | Intervening therapy[†] | Dexamethasone | | | Mechlorethamine | | | Melphalan | | | Carmustine | Doxorubicin | | Vincristine | |
|---|---|---|---|---|---|---|---|---|---|---|---|---|---|---|---|---|
| | | | 0.4 | 1.3 | 4.0 | 0.7 | 1.2 | 3.5 | 2.5 | 4.2 | 12.5 | 11.0 | 1.2 | 1.6 | 0.08 | 0.26 |
| CLL-A | 11/17/83 | — | 26 | | 32 | 77 | 62 | 47 | 90 | 70 | 41 | 44 | 80 | | | |
| CLL-A | 12/14/84 | CLB. CTX | | | | 81 | 83 | 47 | 84 | 96 | 91 | 22 | 70 | | | |
| CLL-A | 02/21/85 | DEX | 72 | | 57 | | | | | | | | | | | |
| CLL-B | 07/03/80 | — | 24 | | | 58 | | 0 | 17 | | 0 | 1 | 9 | | | |
| CLL-B | 11/07/80 | LPAM + P | 43 | | | 86 | | 59 | 59 | | 1 | 1 | 13 | | | |
| CLL-C | 04/10/80 | — | | | | | | 2 | | | 2 | | | | | |
| CLL-C | 10/24/80 | LPAM + P‡ | | | | | | 0 | | | 0 | | 12 | | | |
| CLL-C | 01/05/81 | V + P | | | | | | 0 | | | 9 | | 87 | | | |
| CLL-D | 09/20/84 | — | 4 | | 6 | | | | 59 | | 61 | | | | | |
| CLL-D | 12/14/84 | CLB + P | 108 | | 108 | | | | 115 | | 81 | | | | | |
| CLL-D | 05/16/85 | CLB (no P) | | | 2 | | | | 125 | | 91 | | | | | |
| CLL-E | 10/18/83 | — | 49 | | 47 | 51 | | 5 | 62 | | 19 | | | | | |
| CLL-E | 10/31/83 | — | 44 | | 39 | 80 | | 28 | | | | | | | | |
| CLL-E | 09/21/84 | CLB. COPP | 58 | | 51 | 57 | | 15 | 79 | | 37 | | | | | |
| CLL-J | 09/26/83 | — | 49 | 37 | 43 | 74 | 71 | 63 | 67 | 72 | 39 | 55 | 68 | | | |
| CLL-J | 03/02/84 | CLB | 81 | 77 | 89 | 64 | 95 | 59 | 34 | 58 | 39 | 52 | 58 | | | |
| CLL-K | 03/22/80 | — | | | | | | | | | 10 | | 45 | | | |
| CLL-K | 04/22/80 | CTX | | | | | | | | | 10 | | 78 | | | |
| CLL-M | 02/19/85 | — | 29 | | 17 | 29 | | 3 | 7 | | 5 | | 67 | | | |
| CLL-M | 03/10/86 | (CVP§). CLB. CTX | 15 | | 11 | 69 | | 58 | 76 | | 96 | | 119 | | | |
| MM-A | 04/02/82 | — | 130 | | | 62 | | 17 | 88 | | 15 | 87 | | | | |
| MM-A | 05/13/82 | CTX | 44 | | | 98 | | 91 | 98 | | 37 | 87 | 44 | | | |
| MM-A | 10/21/82 | BCNU + DOX + V | 58 | | | 148 | | | | | 96 | 96 | 84 | | | |
| MM-B | 11/12/80 | — | 73 | | | | | | | | 8 | 22 | | | | |
| MM-B | 09/09/82 | BCNU | 60 | | | | | | | | 56 | 62 | | | | |
| MM-C | 01/15/84 | — | 24 | | 24 | 24 | | | 31 | | 2 | 49 | 5 | | | |
| MM-C | 03/01/84 | LPAM + P | 19 | | 33 | 20 | | | 46 | | 14 | 71 | 15 | | | |
| MM-D | 02/03/81 | — | 51 | | | | | | 85 | | 3 | | | | | |
| MM-D | 06/16/83 | LPAM + P | 55 | | 10 | | | | 70 | | 52 | | | | | |
| MM-D | 11/08/83 | DEX (high dose) | 45 | 100 | 104 | | | | | | | | | | | |
| ALL-A | 01/28/83 | — | 28 | 32 | 27 | 83 | | 13 | | | | | | 21 | 15 | 12 |
| ALL-A | 02/27/84 | V + P + ASP + DOX + CTX | 0 | 1 | 0 | 136 | | 65 | | | | | | 92 | 76 | 50 |
| ALL-B | 07/30/82 | — | | | 0 | | | | | | | | | | | |
| ALL-B | 08/17/84 | V + P + ASP + DAUN + CTX | | | 29 | | | | | | | | | | | |
| ALL-C | 06/30/83 | — | 47 | | 37 | | | | | | | | | | 32 | 18 |
| ALL-C | 10/30/84 | V + P + ASP + DAUN + CTX | 79 | | 79 | | | 29 | | | | | | 47 | 82 | 64 |
| ALL-C | 07/25/85 | A-BM-T | 56 | | 54 | | | 28 | | | | | | | 34 | 44 |
| ALL-C | 08/05/85 | CTX (high dose) | | | | | | 52 | | | | | | | | |
| ALL-D | 10/04/82 | — | 26 | | 16 | | | | | | | | | | | 63 |
| ALL-D | 05/30/85 | V + P + ASP + DAUN | 67 | | 59 | | | | | | | | | | 75 | 82 |
| ALL-D | 02/12/86 | V + P + ASP + DAUN | 82 | | | | | | | | | | | | 72 | |
| ALL-E | 02/23/83 | — | 42 | | 31 | | | | | | | | | | 31 | |
| ALL-E | 06/06/85 | V + P + ASP | 110 | | 98 | | | | | | | | | | 101 | |
| CML-BC | 11/09/82 | — | 61 | | 73 | 114 | | 30 | | | | | | 86 | 69 | 34 |
| CML-BC | 12/14/82 | V + P | 91 | | 68 | 102 | | 35 | | | | | | 59 | 71 | 50 |

[*] Values = % cell survival. Concentrations shown for each drug are μg/ml. See footnote (*) to table 5. Significance levels are based on paired t test calculations from individual paired comparisons of the 1st and 2nd assays in specimens from patients who had at least 1 course of chemotherapy between the times of the 1st and 2nd assays.

[†] CLB = chlorambucil; CTX = cyclophosphamide; DEX = dexamethasone; LPAM = melphalan; P = prenisone; V = vincristine. COPP = cyclophosphamide, vincristine, prednisone, and procarbazine; CVP = cyclophosphamide, vincristine, and prednisone; BCNU = carmustine; DOX = doxorubicin; ASP = L-asparaginase; DAUN = daunorubicin; and A-BM-T = autologous bone marrow transplant.

[‡] 2 cycles only (4/28/80 and 5/24/80).

[§] 1 cycle only (2/85, with partial response).

The average percent cell survival of the first of 104 paired assay comparisons in patients without intervening chemotherapy was 46%, and the average of the second of the paired assay comparisons was 45%. The average percent cell survival of the first of 114 paired assay comparisons in patients with intervening chemotherapy was 45%, and the average of the second of the paired assay comparisons was 61%. Next, we considered only those paired assays in which cells were initially sensitive (cell survival < 30%) to a particular drug at the time of the first assay. The average percent cell survival of the first of 40 paired assay comparisons in patients without intervening chemotherapy was 12%, and the average of the second of the paired assay comparisons was 14%. The average percent cell survival of the first of 47 paired assay comparisons in patients with intervening chemotherapy was 14%, and the average of the second of the paired assay comparisons was 44%.

The above data are not easily analyzed by statistical methods as quantitative continuous variables. However, they can be analyzed as qualitative variables by classifying each assay result as either drug "sensitive" (< 30% cell survival) or "resistant" (<30% cell survival) and comparing the first and second metachronous assays, as shown in Table 7.

TABLE 7.—Summated results of metachronous assays

| | Results of 1st assay/results of 2nd assay* | | | |
| --- | --- | --- | --- | --- |
| | S/S | S/R | R/S | R/R |
| No intervening therapy | 35 | 5 | 3 | 61 |
| Intervening chemotherapy | 19 | 28 | 3 | 64 |

*Values in table = No. of assays. S = sensitive (< 30% cell survival); R = resistant (> 30% cell survival).

These data are noteworthy in several respects. First, in the absence of intervening chemotherapy, there was concordance between the results of the first and second assays in 91% (96 of 105) of instances. Assays initially resistant remained resistant in 95% (61 of 64) of instances. Specimens initially sensitive remained sensitive in 87.5% (35 of 40) of instances. In the presence of chemotherapy, assays initially resistant remained resistant in 96% (64 of 67) of instances. However, assays initially sensitive remained sensitive in only 40% (19 of 47) of instances. Thus, assays initially sensitive were significantly more likely to become resistant in the presence of intervening chemotherapy than in the absence of intervening chemotherapy (P < 0.0001), by chi-Square analysis).

## Claims

1. A method for monitoring acquisition of drug resistance of tumor cells in patients subjected to chemotherapeutic treatment, which method comprises
periodically withdrawing fresh tumor cells from said subject, and
determining the sensitivity of said cells to the administered drug in vitro.

2. The method of claim 1 wherein the tumor cells are assayed in vitro by determining their ability to exclude a dye excluded by living cells after incubation with the drug.

3. A method to successively modify a chemotherapeutic protocol in a human subject which comprises
periodically withdrawing fresh tumor cells from the subject;
determining the sensitivity of said cells to the administered drug in vitro;
comparing the sensitivity of the later-withdrawn cells to that of earlier withdrawn cells: and if diminished sensitivity is found,
determining the sensitivity of said cells to alternative drugs or combinations thereof.

4. A method to diagnose relapse in a patient receiving chemotherapy which comprises
withdrawing fresh tumor cells from said subject, and
determining the sensitivity of said cells to the administered drug in vitro.

FIG. I

n=43,26
p<.0001

RESPONDERS    NON-RESPONDERS    ■ RESPONDERS
                                 □ NON-RESPONDERS

Prospective clinical correlations between in vitro and in vivo chemosensitivity in patients with CLL. ALL. multiple myeloma. non-Hodgkin's lymphomas. and CML in blast crisis (for CML. only patients treated with vincristine and prednisone are included). The ordinate depicts % cell survival in vitro (30% is the dividing line between sensitive and resistant). Symbols indicate the lowest % cell survival in vitro among the agents tested which were ultimately incorporated into the treatment regimen. For these clinical correlation studies. a single in vitro concentration was utilized for each drug. Three drugs were tested using a 1-hr exposure time. These included ( g/ml): mechlorethamine. 3.5: melphalan. 12.5: and doxorubicin. 1.2 (for CLL. multiple myeloma. and non-Hodgkin's lymphoma) or 1.67 (for ALL). Mechlorethamine was used as the index alkylating agent to predict for cyclophosphamide and chlorambucil. Doxorubicin was also used to predict for daunorubicin. Two drugs were tested using a continuous 4-day exposure time. These included: vincristine. 0.08: and dexamethasone. 0.4. Dexamethasone was used to predict for prednisone. L-Asaraginase was not tested in vitro. but most ALL patients received induction treatment regimens containing this agent. along with vincristine. dexamethasone. and sometimes an anthracycline. CLL patients were largely treated with chlorambucil with or without prednisone. Untreated myeloma patients were treated with melphalan/prednisone. and relapsed patients received doxorubicin. carmustine. prednisone. and vincristine. Non-Hodgkin's lymphoma patients received standard regimens including CVP (cyclophosphamide. vincristine. and prednisone). CHOP (cyclophosphamide. doxorubicin. vincristine. and prednisone). and COPP (cyclophosphamide. vincristine. prednisone. and procarbazine). CML-blast crisis patients all received vincristine and prednisone. and 2 patients also received doxorubicin. The bar graphs on the far right depict the average (mean) % cell survival for responders and nonresponders. n=Nos. of responders and nonresponders. respectively. whose assays are represented.

FIG. 2

FIG. 3